# EUROPÄISCHE PATENTSCHRIFT

(11) **EP 0 806 021 B1**
(45) Veröffentlichungstag und Bekanntmachung des Hinweises auf die Patenterteilung: **24.01.2001**
(21) Anmeldenummer: 96939908.8
(22) Anmeldetag: 22.11.1996
(51) Int. Cl.: G07D 7/00

(54) **VORRICHTUNG UND VERFAHREN ZUR BESTIMMUNG DER STEIFIGKEIT VON BLATTGUT, WIE Z.B. BANKNOTEN**
DEVICE AND PROCESS FOR DETERMINING THE STIFFNESS OF SHEET-LIKE ARTICLES SUCH AS BANK NOTES
DISPOSITIF ET PROCEDE POUR DETERMINER LA RIGIDITE D'UN PRODUIT EN FEUILLE TEL QUE DES BILLETS DE BANQUE

(30) Priorität: 23.11.1995 DE 19543674
(43) Veröffentlichungstag der Anmeldung: 12.11.1997
(73) Patentinhaber: Giesecke & Devrient GmbH, 81677 München (DE)
(72) Erfinder: WUNDERER, Bernd, D-80805 München (DE); SCHANDA, Ulrich, D-83607 Holzkirchen (DE)
(74) Vertreter: Klunker . Schmitt-Nilson . Hirsch
(86) Internationale Anmeldenummer: EP9605175
(87) Internationale Veröffentlichungsnummer: WO9719425

(56) Entgegenhaltungen:
- EP-A- 0 073 133
- EP-A- 0 357 406
- EP-A- 0 521 625
- US-A- 4 446 735
- US-A- 4 519 249

## Beschreibung

Die Erfindung betrifft eine Vorrichtung und ein Verfahren zur Bestimmung der Steifigkeit von Blattgut, wie z. B. Banknoten. .

Eine solche Vorrichtung ist aus der EP-A-00 73 133 und US-A-4 519 249 bekannt. Bei dieser Vorrichtung wird das Blattgut zwischen zwei Flachriemen in der Mitte geführt und über eine doppelkonische Rolle um einen Winkel von 180° umgelenkt. Dabei findet gleichzeitig in Längs- und in Querrichtung eine Deformation des Blattguts statt. Die bei diesem Vorgang erzeugten Geräusche werden von einem Mikrofon detektiert. Aus den detektierten Geräuschen bestimmt dann eine Auswerteeinrichtung die Steifigkeit des Blattguts.

Ein Nachteil der Vorrichtung besteht darin, daß die erzeugten Geräusche stark abnehmen, wenn die Steifigkeit eines Blattes mehrfach gemessen wird. Die Steifigkeit des Blattgutes wird bei jedem Meßvorgang durch die mit dem Meßvorgang verbundene Deformation geringer.

Ausgehend davon liegt der Erfindung die Aufgabe zugrunde, eine Vorrichtung bzw. ein Verfahren zur Bestimmung der Steifigkeit von Blattgut vorzuschlagen, bei der die Steifigkeit des Blattguts im wesentlichen unverändert bleibt.

Diese Aufgabe wird durch die Merkmale des Hauptanspruchs gelöst.

Der Grundgedanke der Erfindung besteht im wesentlichen darin, zur Erzeugung von Geräuschen mechanische Mittel vorzusehen, die das Blattgut periodisch berühren und es so zu Schwingungen anregen. Die durch die Schwingungen erzeugten Geräusche werden dann von einem Detektor detektiert. Aus den detektierten Geräuschen bestimmt dann eine Auswerteeinrichtung die Steifigkeit des Blattguts. Da die Lautstärke der erzeugten Geräusche hierbei annähernd proportional zur Steifigkeit des Blattgutes ist, stellt die Lautstärke der erzeugten Geräusche ein direktes Maß für Steifigkeit des Blattgutes dar.

Das Blattgut ist derart in einer Transporteinrichtung gehalten bzw. geführt, daß es im Bereich der Berührung in gewissen Grenzen der mechanischen Einwirkung nachgeben kann, ohne irreversibel deformiert zu werden.

Ein Vorteil der Erfindung besteht darin, daß die Steifigkeit des Blattguts nahezu unverändert bleibt. Auch bei mehrfachen Bestimmungen der Steifigkeit eines Blattguts bleibt die Intensität der erzeugten Geräusche im wesentlichen gleich. Die Reproduzierbarkeit der Intensität der erzeugten Geräusche beträgt im Mittel mindestens 95%.

In einer bevorzugten Ausführungsform der Erfindung weisen die mechanischen Mittel eine rotierende Walze mit einer bestimmten Anzahl von Ecken auf. Die Ecken sind rotationssymmetrisch auf der Walze angeordnet. Das Blattgut wird von einer Transporteinrichtung durch die Vorrichtung transportiert und die Antriebsachse der rotierenden Walze senkrecht zur Transportrichtung des Blattgutes angeordnet. Die Drehrichtung der rotierenden Walze ist in Richtung der Transportrichtung gerichtet. Die Drehfrequenz der rotierenden Walze wird so gewählt, daß die von der Drehfrequenz und vom Radius der Walze abhängige Umfangsgeschwindigkeit größer oder gleich der Transportgeschwindigkeit des Blattguts ist.

Weitere Merkmale der Erfindung ergeben sich aus den Unteransprüchen. Nachfolgend werden verschiedene Ausführungsformen der Erfindung anhand der Figuren beschrieben. Es zeigen:
- Fig. 1: Prinzipskizze einer bevorzugten Ausführungsform,
- Fig. 2: Prinzipskizze der bevorzugte Ausführungsform mit Transportriemen,
- Fig. 3: Prinzipskizze von unten gegen die bevorzugte Ausführungsform mit Transportriemen,
- Fig. 4: Prinzipskizze von unten gegen die bevorzugte Ausführungsform mit Transportriemen und Leitplatte,
- Fig. 5: Prinzipskizze einer Variation der bevorzugten Ausführungsform,
- Fig. 6: Prinzipskizze einer zweiten Ausführungsform der Erfindung,
- Fig. 7: Prinzipskizze einer dritten Ausführungsform der Erfindung,
- Fig. 8: Prinzipskizze einer vierten Ausführungsform der Erfindung,
- Fig. 9: Prinzipskizze einer Weiterbildung der vierten Ausführungsform der Erfindung,
- Fig. 10: Prinzipskizze einer fünften Ausführungsform der Erfindung.

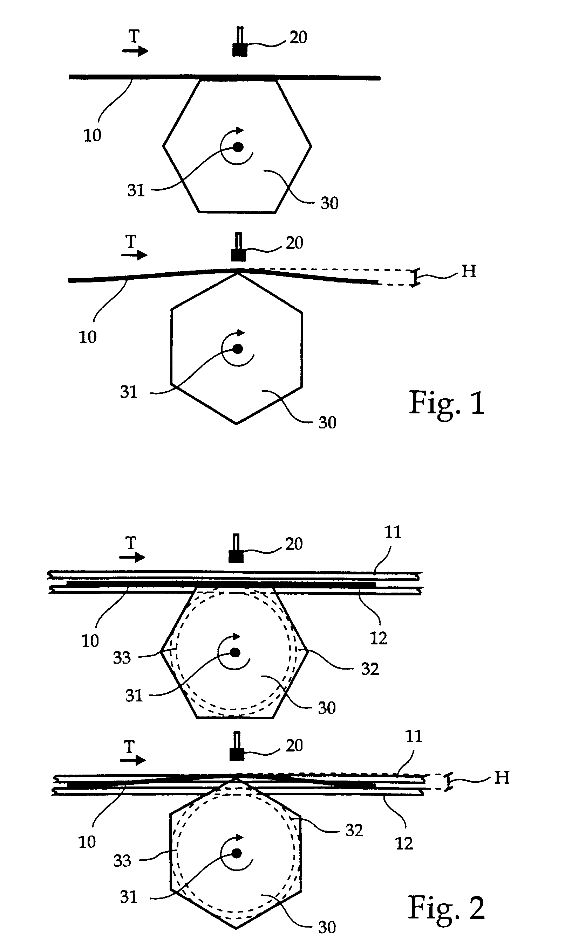

Die Fig. 1 zeigt eine Prinzipskizze einer bevorzugten Ausführungsform der Erfindung in einer Seitenansicht. Das Blattgut 10 wird beispielsweise mittels Transportriemen, die aus Gründen der Übersicht hier zunächst nicht dargestellt sind, in Richtung der Transportrichtung T durch die Vorrichtung transportiert. Als mechanisches Mittel zur Erzeugung von Geräuschen ist hier eine rotierende Walze 30 mit sechs rotationssymmetrisch angeordneten Ecken dargestellt. Die Antriebsachse 31 der rotierenden Walze 30 ist senkrecht zur Transportrichtung T des Blattguts ausgerichtet.

Die Drehrichtung der Walze 30 ist in Richtung der Transportrichtung T des Blattguts gerichtet. Die Drehfrequenz der Walze 30 wird so gewählt, daß die von der Drehfrequenz und vom Radius der Walze 30 abhängige Umfangsgeschwindigkeit der Walze 30 größer oder gleich der Transportgeschwindigkeit des Blattguts ist. Durch diese Wahl von Drehfrequenz und Drehrichtung kann verhindert werden, daß das durch die Vorrichtung transportierte Blattgut an eine Walzenecke anstoßen kann und dadurch abgebremst wird. Ein solches Abbremsen würde die Staugefahr im Transportsystem vergrößern. Prinzipiell kann die Funktionalität der Vorrichtung auch bei geänderter Drehrichtung bzw. Drehfrequenz der Walze 30 gewährleistet werden.

Durch die Rotation der Walze 30 berühren die Ecken periodisch das Blattgut 10 und regen es so zu Schwingungen an. Das Blattgut wird dabei im Bereich der Walze 30 um eine Hubhöhe H angehoben. Die Hubhöhe H ist ein Maß für die Lautstärke der erzeugten Geräusche.

Der Radius der Walze 30, die Anzahl N der Ecken und die Drehfrequzenz der Walze 30 sind die Parameter, mittels derer die bevorzugte Ausführungsform der Erfindung optimal an die jeweiligen apparativen Randbedingungen, wie z.B. die maximale räumliche Ausdehnung der Vorrichtung, angepaßt werden kann. Die Berührungsfrequenz der Walze auf das Blattgut 10 ergibt sich aus dem Produkt der Drehfrequenz und der Eckenzahl. Die Hubhöhe H hängt von der Wahl des Radius der Walze und der Eckenzahl ab.

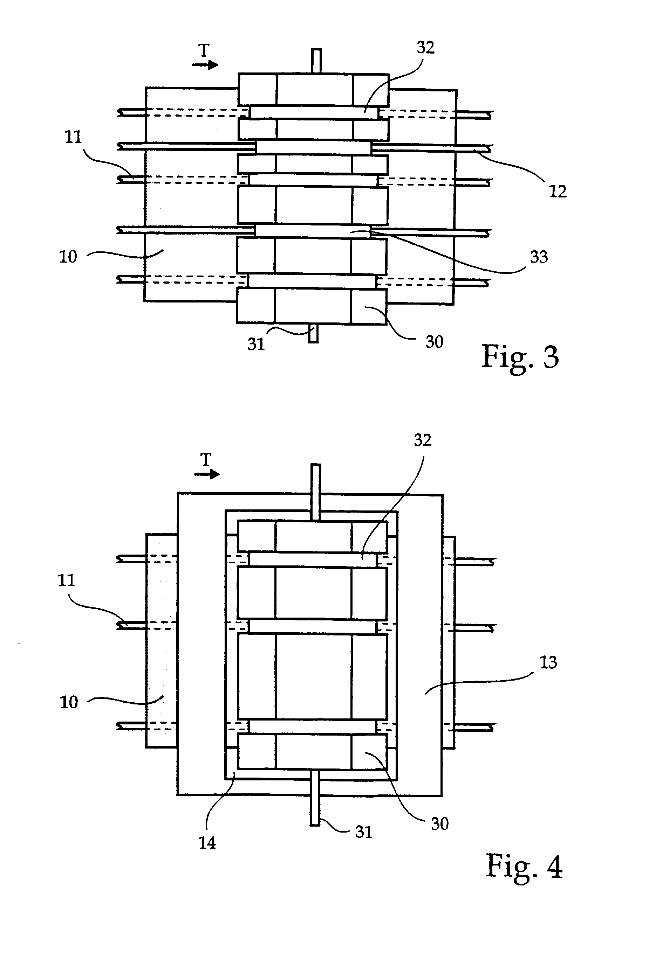

Die Fig. 2 und Fig. 3 zeigen die bevorzugte Ausführungsform mit einer Transporteinrichtung in einer Seitenansicht und von unten gesehen, wobei die Transporteinrichtung in die sechseckige Walze 30 integriert ist. Die Transporteinrichtung weist drei obere Transportriemen 11 und zwei untere Transportriemen 12 auf, zwischen denen das Blattgut 10 zum Transport durch die Vorrichtung eingeklemmt ist.

Um Wechselwirkungen zwischen der rotierenden Walze 30 und den oberen Transportriemen 11 bzw. unteren Transportriemen 12 zu vermeiden, sind in der Walze freilaufende Rollen 32, 33 vorgesehen, über die die entsprechenden Transportriemen geführt werden. Diese Rollen lassen sich von der Antriebsachse 31 beispielsweise durch entsprechende Kugellager entkoppeln, so daß die Transportriemen 11 bzw. 12 unabhängig von der Umfangsgeschwindigkeit der Walze 30 mit konstanter Transportgeschwindigkeit bewegt werden können. Der Radius der freilaufenden Rollen 32 wird bevorzugt gleich dem Radius des Inkreises der Walze 30 gewählt. Der Radius der freilaufenden Rollen 33 wird bevorzugt kleiner als der Radius des Inkreises der sechseckigen Walze 30 gewählt. Ist kein Blattgut 10 anwesend, so schließen die oberen Kanten der unteren Transportriemen 12 und die unteren Kanten der oberen Transportriemen 11 in der Position der Walze 30 gemäß des oberen Teils der Fig. 1 mit der Walze 30 ab. Die Berührung der Walze 30 mit dem Blattgut 10 findet in den Bereichen zwischen den Transportriemen 11 und 12 statt.

In Fig. 4 ist die bevorzugte Ausführungsform mit oberen Transportriemen 11 und einer Leitplatte 13 von unten gesehen dargestellt. Die Leitplatte 13 ersetzt die unteren Transportriemen 12 gemäß Fig. 3 in ihrer Funktion. Beim Transport durch die Vorrichtung wird das Blattgut 10 von den oberen Transportriemen 11 über die Leitplatte 13 transportiert. Auch hier sind die Transportriemen 11 durch freilaufende Rollen 32 von der Walze 30 entkoppelt. In der Leitplatte 13 ist eine Aussparung 14 vorgesehen, durch die die Walze 30 mit dem Blattgut 10 in Kontakt tritt.

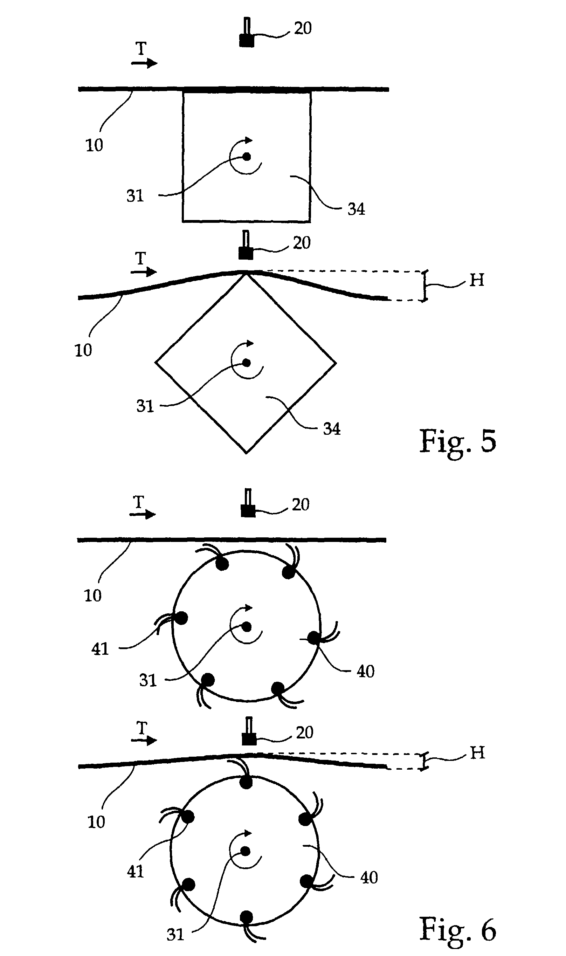

Die Fig. 5 zeigt eine Variation der bevorzugten Ausführungsform mit einer viereckigen Walze 34. Im Gegensatz zur in Fig. 1 dargestellten sechseckigen Walze 30 ist die Hubhöhe H der viereckigen Walze 34 größer als die Hubhöhe H der sechseckigen Walze 30. Bei Konstanz der anderen Parameter sind die von der viereckigen Walze 34 erzeugten Geräusche somit lauter als die von der sechseckigen Walze 30. Um bei beiden Varianten der Ausführungsform eine konstante Berührungsfrequenz zu gewährleisten, muß die Drehfrequenz der viereckigen Walze 34 gegenüber der sechseckigen Walze 30 entsprechend erhöht werden. Die Drehfrequenz läßt sich jedoch aufgrund der bei hohen Drehfrequenzen auftretenden mechanischen Probleme nur in gewissen Grenzen variieren, so daß eine Optimierung der Parameter notwendig wird, um bei einer vorgegebenen Berührungsfrequenz hinreichend laute Geräusche zu erzeugen.

Die Fig. 6 zeigt eine zweite Ausführungsform der Erfindung, bei der auf einer rotierenden Walze 40 Bürsten 41 rotationssymmetrisch angeordnet sind. Bevorzugt werden diese Bürsten 41 jeweils auf einer eigenen Achse beweglich ausgeführt, so daß die Bürsten 41 durch die bei der Rotation der Walze 40 entstehende Zentrifugalkraft nach außen ausklappen. Die zur Bestimmung der Steifigkeit des Blattguts notwendigen Geräusche werden hier durch das periodische Berühren der Bürsten 41 gegen das Blattgut 10 erzeugt. Die zur bevorzugten Ausführungsform der Erfindung beschriebenen Parameter, sowie die dargestellten Variationen lassen sich leicht auf diese Ausführungsform übertragen, so daß hier auf eine erneute Beschreibung verzichtet werden kann.

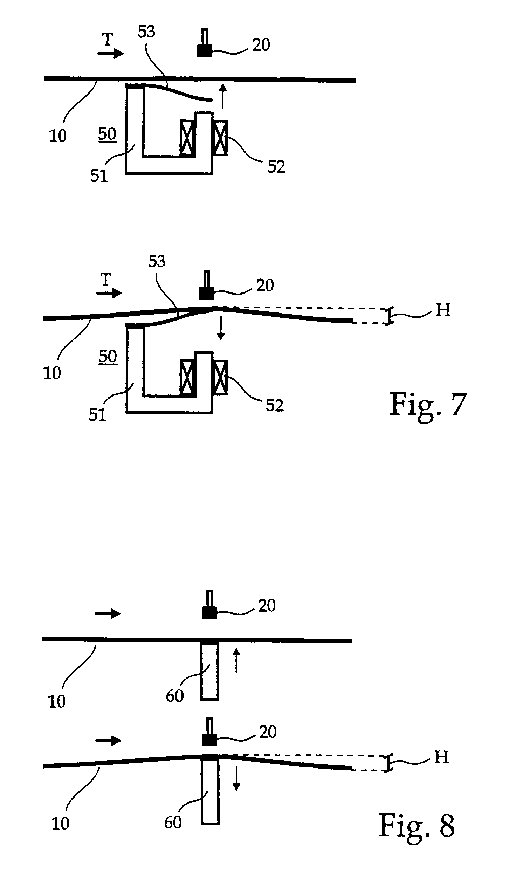

Die Fig. 7 zeigt eine dritte Ausführungsform der Erfindung, bei der die Einrichtung zur Erzeugung von Geräuschen als mechanisches Mittel einen Elektromagneten 50 mit einem Joch 51, einer Spule 52 und einer beweglichen Zunge 53 aufweist. Durch Anlegen einer Wechselspannung an die Spule 50 wird die bewegliche Zunge 53 in Schwingungen versetzt. Die Geräusche werden durch periodisches Berühren des Blattguts 10 durch die Zunge 53 erzeugt. Die Schwingungsfrequenz der beweglichen Zunge 53 entspricht dabei der Frequenz der angelegten Wechselspannung. Die Hubhöhe H kann durch die maximale Spannungsdifferenz der Wechselspannung verändert werden.

Die Fig. 8 zeigt eine vierte Ausführungsform der Erfindung, bei der das mechanische Mittel zur Erzeugung von Geräuschen ein Piezoelement 60 aufweist. Durch Anlegen einer Wechselspannung kann die räumliche Ausdehnung des Piezoelements 60 in der Richtung senkrecht zur Blattgutebene verändert werden. Die Frequenz der Ausdehnung des Piezoelements 60 entspricht der Frequenz der angelegten Wechselspannung. Das Maß der Ausdehnung des Piezoelements 60 hängt von der maximalen Spannungsdifferenz der Wechselspannung ab, ist jedoch relativ gering. Dies führt lediglich zu einer geringen Hubhöhe H der Blattguts 10 und somit auch zu einer geringen Lautstärke der erzeugten Geräusche.

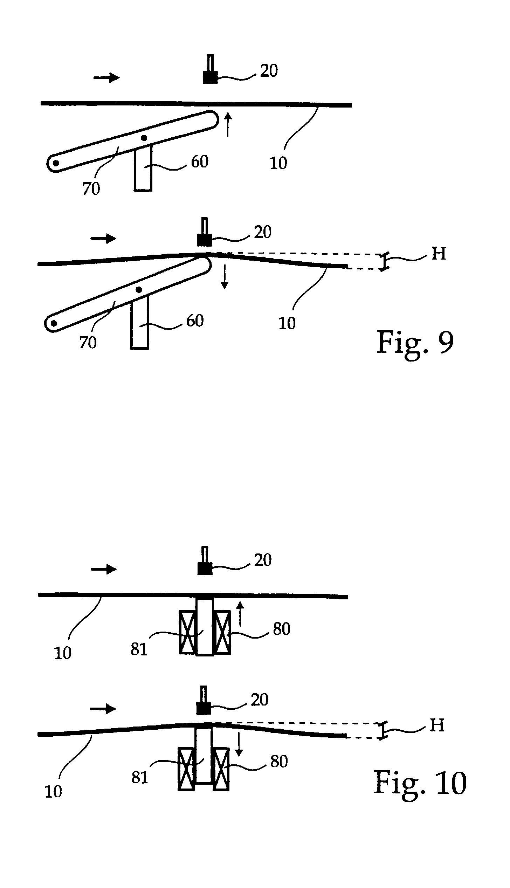

Eine Methode zur Vergrößerung der Hubhöhe H des Blattguts 10 ist in Fig. 9 gezeigt. Sie besteht darin, ein zusätzliches Hebelsystem 70 vorzusehen, das mit dem Piezoelement 60 verbunden ist und mittels dem die Ausdehnung des Piezoelements 60 so verstärkt wird, daß eine gewünschte Hubhöhe H erreicht wird.

Alternativ dazu können beispielsweise sogenannte bimorphe Piezoelemente 60 eingesetzt werden, um die Hubhöhe H des Blattguts 10 zu vergrößern. Bei solchen bimorphen Piezoelementen 60 stehen mindestens zwei Piezoelemente 60 in einem festen Verbund miteinander und die Ausdehnung der Piezoelemente 60 wird in eine Biegung der Piezoelemente 60 umgesetzt. Mit diesen bimorphen Piezoelementen 60 lassen sich Hubhöhen H von mehreren Millimetern erzeugen.

Die Fig. 10 zeigt eine fünfte Ausführungsform der Erfindung, bei der die mechanischen Mittel zur Erzeugung der Geräusche eine Spule 80 mit einem beweglichen magnetischen Kern 81 aufweisen. Legt man an die Spule eine Wechselspannung, so schwingt der bewegliche magnetische Kern 81 mit der Frequenz der Wechselspannung. Die Geräusche entstehen durch periodisches Berühren des Blattguts 10 durch den magnetischen Kern 81. Die Auslenkung des beweglichen Kerns 81 und somit die Hubhöhe H des Blattguts 10 kann durch die maximale Spannungsdifferenz der Wechselspannung beeinflußt werden.

Zusätzlich zu den oben beschriebenen Ausführungsformen ist es dem Fachmann in Kenntnis des erfinderischen Grundgedankens selbstverständlich möglich, Variationen der beschriebenen Ausführungsformen oder neue Ausführungsformen zu entwickeln, die auf dem erfinderischen Grundgedanken der Erfindung beruhen. Speziell ist eine analoge Übertragung der zur bevorzugten Ausführungsform erläuterten Transporteinrichtungen auf die anderen Ausführungsformen möglich.

Zur Auswertung der detektierten Geräusche können alle bekannten Methoden der analogen bzw. digitalen Signalverarbeitung verwendet werden.

## Patentansprüche

1. Vorrichtung zur Bestimmung der Steifigkeit von Blattgut, wie z. B. Banknoten, mit
- einer Einrichtung zur Erzeugung von Geräuschen unter Verwendung des Blattguts,
- einem Detektor zur Detektion der erzeugten Geräusche,
- einer Auswerteeinrichtung, die aus den detektierten Geräuschen die Steifigkeit des Blattguts bestimmt,
dadurch **gekennzeichnet**, daß die Einrichtung zur Erzeugung von Geräuschen mechanische Mittel (30, 34, 40, 41, 50, 60, 70, 80, 81) aufweist, die das Blattgut (10) periodisch berühren und es so zu Schwingungen anregen.

2. Vorrichtung nach Anspruch 1, dadurch **gekennzeichnet**, daß die mechanischen Mittel eine rotierende Walze (30, 34) mit einer bestimmten Anzahl (N) von rotationssymmetrisch angeordneten Ecken aufweisen.

3. Vorrichtung nach Anspruch 1, dadurch **gekennzeichnet**, daß die mechanischen Mittel eine rotierende Walze (40) aufweisen, an der rotationssymrnetrisch Bürsten (41) angeordnet sind.

4. Vorrichtung nach Anspruch 2 oder 3, dadurch **gekennzeichnet**, daß das Blattgut mittels einer Transporteinrichtung (11,12) durch die Vorrichtung transportiert wird und die Antriebsachse (31) der rotierenden Walze (30, 34, 40) senkrecht zur Transportrichtung (T) des Blattguts angeordnet ist.

5. Vorrichtung nach Anspruch 4, dadurch **gekennzeichnet**, daß die Umfangsgeschwindigkeit der Walze (30, 34, 40) in Richtung der Transportrichtung (T) des Blattguts gerichtet und größer oder gleich der Transportgeschwindigkeit des Blattguts ist.

6. Vorrichtung nach Anspruch 4, dadurch **gekennzeichnet**, daß die Transporteinrichtung obere und/oder untere Transportriemen (11,12) aufweist, die durch freilaufende Rollen (32, 33) von der Antriebsachse (31) entkoppelt sind.

7. Vorrichtung nach Anspruch 6, dadurch **gekennzeichnet,** daß die Transporteinrichtung eine Leitplatte (13) mit einer Aussparung (14) aufweist, durch die die Walze (30, 34, 40) in Kontakt mit dem Blattgut (10) tritt.

8. Vorrichtung nach Anspruch 1, dadurch **gekennzeichnet**, daß die mechanischen Mittel einen Elektromagneten (50) mit einer beweglichen Zunge (53) aufweisen.

9. Vorrichtung nach Anspruch 1, dadurch **gekennzeichnet**, daß die mechanischen Mittel ein Piezoelement (60) aufweisen.

10. Vorrichtung nach Anspruch 9, dadurch **gekennzeichnet**, daß die mechanischen Mittel ein zusätzliches Hebelsystem (70) aufweisen, das mit dem Piezoelement (60) verbunden ist.

11. Vorrichtung nach Anspruch 9, dadurch **gekennzeichnet**, daß die mechanischen Mittel mindestens ein weiteres Piezoelement (60) aufweisen und daß die Piezoelemente (60) in einem festen Verbund miteinander stehen, so daß die Ausdehnung der Piezoelemente (60) in eine Biegung der Piezoelemente umgesetzt wird.

12. Vorrichtung nach Anspruch 1, dadurch gekennzeichnet, daß die mechanischen Mittel eine Spule (80) mit einem magnetischen Kern (81) aufweisen.

13. Verfahren zur Bestimmung der Steifigkeit von Blattgut, wie z. B. Banknoten, bei dem
- unter Verwendung des Blattguts Geräusche erzeugt werden,
- die erzeugten Geräusche des Blattguts detektiert werden und
- aus den detektierten Geräuschen des Blattguts die Steifigkeit des Blattguts bestimmt wird
dadurch **gekennzeichnet,** daß zur Erzeugung der Geräusche, das Blattgut (10) durch periodisches Berühren des Blattguts (10) zu Schwingungen angeregt wird.

## Claims

1. An apparatus for determining the stiffness of sheet material such as bank notes, comprising
- a device for producing sounds using the sheet material,
- a detector for detecting the produced sounds,
- an evaluating device for determining the stiffness of the sheet material from the detected sounds,
characterized in that the device for producing sounds has mechanical means (30, 34, 40, 41, 50, 60, 70, 80, 81) which periodically touch the sheet material (10), causing it to vibrate.

2. An apparatus according to claim 1, characterized in that the mechanical means have a rotating roll (30, 34) with a certain number (N) of rotationally symmetric corners.

3. An apparatus according to claim 1, characterized in that the mechanical means have a rotating roll (40) with brushes (41) disposed in rotational symmetry thereon.

4. An apparatus according to claim 2 or 3, characterized in that the sheet material is transported through the apparatus by means of a transport device (11, 12), and the driving axle (31) of the rotating roll (30, 34, 40) is disposed perpendicular to the transport direction (T) of the sheet material.

5. An apparatus according to claim 4, characterized in that the circumferential speed of the roll (30, 34, 40) is in the direction of transport (T) of the sheet material and is greater than or equal to the transport speed of the sheet material.

6. An apparatus according to claim 4, characterized in that the transport device has upper and/or lower transport belts (11, 12) decoupled from the driving axle (31) by free-runing rollers (32, 33).

7. An apparatus according to claim 6, characterized in that the transport device has a guiding plate (13) with a gap (14) through which the roll (30, 34, 40) comes in contact with the sheet material (10).

8. An apparatus according to claim 1, characterized in that the mechanical means have an electromagnet (50) with a movable tongue (53).

9. An apparatus according to claim 1, characterized in that the mechanical means have a piezoelectric element (60).

10. An apparatus according to claim 9, characterized in that the mechanical means have an additional lever system (70) connected with the piezoelectric element (60).

11. An apparatus according to claim 9, characterized in that the mechanical means have at least one further piezoelectric element (60), and the piezoelectric elements (60) are firmly interconnected so that the expansion of the piezoelectric elements (60) is converted into a bending of the piezoelectric elements.

12. An apparatus according to claim 1, characterized in that the mechanical means have a coil (80) with a magnetic core (81).

13. A method for determining the stiffness of sheet material such as bank notes, wherein
- sounds are produced using the sheet material,
- the produced sounds of the sheet material are detected, and
- the stiffness of the sheet material is determined from the detected sounds of the sheet material,
characterized in that for producing the sounds the sheet material (10) is caused to vibrate by periodic touching of the sheet material (10).

## Revendications

1. Dispositif pour déterminer la rigidité d'un produit en feuilles, comme par exemple des billets de banque, avec
- un organe pour l'émission de bruits en utilisant le produit en feuilles,
- un détecteur pour la détection des bruits émis,
- un organe d'évaluation qui détermine la rigidité du produit en feuilles à partir des bruits détectés,
caractérisé en ce que l'organe pour l'émission de bruits comprend des moyens mécaniques (30, 34, 40, 41, 50, 60, 70, 80, 81), qui viennent en contact périodiquement avec le produit en feuilles (10) en le stimulant pour le mettre en vibration.

2. Dispositif selon la revendication 1, caractérisé en ce que les moyens mécaniques comprennent un tambour tournant (30, 34) avec un certain nombre (N) d'arêtes disposées en symétrie de révolution.

3. Dispositif selon la revendication 1, caractérisé en ce que les moyens mécaniques comprennent un tambour tournant (40) sur lequel des brosses (41) sont disposées en symétrie de révolution.

4. Dispositif selon la revendication 2 ou 3, caractérisé en ce que le produit en feuilles est transporté à travers le dispositif par un organe de transport (11, 12) et en ce que l'arbre d'entraînement (31) du tambour tournant (30, 34, 40) est disposé perpendiculairement à la direction de transport (T) du produit en feuilles.

5. Dispositif selon la revendication 4. caractérisé en ce que le tambour (30, 34, 40) tourne dans la direction de transport (T) du produit en feuilles et en ce que sa vitesse périphérique est supérieure ou égale à la vitesse de transport du produit en feuilles.

6. Dispositif selon la revendication 4, caractérisé en ce que l'organe de transport comprend des courroies de transport supérieures et/ou inférieures (11, 12) qui sont découplées de l'arbre d'entraînement (31) par des rouleaux (32, 33) tournant librement.

7. Dispositif selon la revendication 6, caractérisé en ce que l'organe de transport comprend une plaque de guidage (13) avec un évidemment (14) à travers lequel le tambour (30, 34, 40) entre en contact avec le produit en feuilles (10).

8. Dispositif selon la revendication 1, caractérisé en ce que les moyens mécaniques comprennent un électro-aimant (50) avec une languette mobile (53).

9. Dispositif selon la revendication 1, caractérisé en ce que les moyens mécaniques comprennent un élément piézo-électrique (60).

10. Dispositif selon la revendication 9, caractérisé en ce que les moyens mécaniques comprennent un système de levier supplémentaire (70) qui est relié à l'élément piézo-électrique (60).

11. Dispositif selon la revendication 9, caractérisé en ce que les moyens mécaniques comprennent au moins un autre élément piézo-électrique (60) et en ce que les éléments piézo-électriques (60) sont reliés rigidement entre eux, de sorte que l'extension des éléments piézo-électriques (60) soit transformée en une flexion des éléments piézo-électriques.

12. Dispositif selon la revendication 1, caractérisé en ce que les moyens mécaniques comprennent une bobine (80) avec un noyau magnétique (81).

13. Procédé pour déterminer la rigidité d'un produit en feuilles, comme par exemple des billets de banque, dans lequel
- des bruits sont émis en utilisant le produit en feuilles.
- les bruits émis par le produit en feuilles sont détectés, et
- la rigidité du produit en feuilles est déterminée à partir des bruits du produit en feuilles détectés.
caractérisé en ce que, pour l'émission des bruits, le produit en feuilles (10) est stimulé pour le mettre en vibration par des contacts périodiques avec le produit en feuilles (10).
